Europäisches Patentamt

European Patent Office

Office européen des brevets

(11) Publication number: **0 405 907 A1**

# EUROPEAN PATENT APPLICATION

(21) Application number: 90306964.9

(51) Int. Cl.⁵: **A61M 5/14**

(22) Date of filing: 26.06.90

(30) Priority: 30.06.89 GB 8915066
11.12.89 GB 8927917
13.02.90 GB 9003163

(43) Date of publication of application:
02.01.91 Bulletin 91/01

(84) Designated Contracting States:
AT BE CH DE DK ES FR GB GR IT LI LU NL SE

(71) Applicant: Ainsworth, Quentin Paul
139 Dore Road, Dore
Sheffield, Yorkshire(GB)

(72) Inventor: Ainsworth, Quentin Paul
139 Dore Road, Dore
Sheffield, Yorkshire(GB)

(74) Representative: Heath, Peter William Murray
FRY HEATH & CO. St. Georges House 6
Yattendon Roadoad
Horley Surrey RH6 7BS(GB)

(54) Intravascular infusion apparatus.

(57) The invention provides apparatus for intravascular infusion comprising an assembly of a plurality of hollow input connection units (5), and at least one hollow output connection unit (9), the input units (5) comprising hollow members each having an entry (4) extending from infusion tubing (1) or a similar input line, and leading via a flexible cross-connector tube unit (6) terminating in an output end carrying a coupling element (12), the at least one output unit (9) comprising a hollow member having at least one inlet port (10), such inlet port (10) being provided with coupling elements for connection to the coupling elements (12) at the outlet ends of the cross-connector units (6) and the or at least one output unit having (9) an outlet port (14) provided with coupling elements (15) for connection to a cannula, or similar output line (17) whereby the input units (5) may be connected by means of their cross-connector units (6) to the at least one inlet port (10) of the at least one output unit (9).

FIG.1.

## INTRAVASCULAR INFUSION APPARATUS

This invention relates to apparatus for medical intravascular therapy.

Intravascular such as intravenous therapy and monitoring is commonplace in medical practice, especially in Intensive Care situations, and often involves the insertion of a plurality of cannulae into one or more central or peripheral veins. Such cannulae each connect via appropriate couplings to further medical equipment, such as infusion delivery equipment which connects to the cannula (and the patient) by a length of tubing, for the administration of therapy, or from monitoring apparatus.

For the sake of convenience medical devices comprising lumina, cannula, tubing, electrical leads and other elongate medical devices are herein at times described for simplicity as "lines".

Connection between infusion tubing and cannulae commonly incorporate a quick-connect and quick-release leak-proof coupling of the kind known as a "luer-lock" coupling, the male portion of a coupling being incorporated into the patient end of the infusion tubing, and a female portion being incorporated into the external end of each of the cannula.

In order to increase the number of available infusion portals without increasing the number of insertion devices, it has been proposed that a cannula contains more than one lumen (or small bore hollow tube) each of which extends outside the patient via separated tubes each to its own coupling device for connection to an infusion or similar device.

In addition, it is possible, when the desired number of infusions exceeds the available number of cannulae, or the available number of lumina leading to cannulae with multiple lumina therewithin, to utilise apparatus (already proposed) for connection between a cannula and infusion equipment which essentially comprises a manifold device having an outlet carrying a male coupling element for connecting (usually) to the female coupling element on extension tubing, in turn connecting to the cannula, the manifold being hollow and having along its length a plurality of side ports each having a coupling element and capable of connection to the male coupling element from infusion tubing.

With either of these arrangements previously proposed, and in particular the use of the manifold device, the "branching" of infusion tubing away from each cannula does tend in practice to cause difficulty in identifying which tube is carrying which infusion and can be a source of error.

Additionally, it is to be noted in use that an admixture of drugs flowing into the same cannula is chosen such that they are chemically compatible and do not interfere with each other. When drugs are changed during the therapy of a patient, the infusion combinations into each cannula may need to be changed so as to ensure continuing compatibility of the new mixture of drugs within any one cannula. This can well entail the rearrangement of infusion connections leading to the cannula, and again increases the risk of error, and additionally, the risk of the introduction of infection into the infusion line.

It is an object of the present invention to overcome or at least substantially reduce the above-mentioned problems and disadvantages.

In accordance with one aspect of the present invention, there is provided apparatus for intravascular infusion comprising an assembly of a plurality of input connection units, and at least one hollow output connection unit, the input units comprising a hollow members each having an entry extending from infusion tubing or a similar input line, and leading via a flexible cross-connector tube unit terminating in an output end carrying a coupling element, the at least one output unit each comprising a hollow member having at least one inlet port, such inlet port being provided with coupling elements for connection to the coupling elements at the outlet ends of the cross-connector units and the or at least one output unit having an outlet port provided with coupling elements for connection to a cannula, or similar output line whereby the input units may be connected by means of their cross-connector units to the at least one inlet port of the at least one output unit.

In accordance with another aspect of the present invention, there is provided apparatus for intravascular infusion comprising a plurality of hollow input connection units, and a plurality of hollow output connection units, at least some of the input units having an entry port carrying coupling elements for connection to infusion tubing or a similar input line, and at least some of said input connection units linking directly or indirectly to a flexible cross-connector tube unit, at least some of the cross-connector tube units terminating in coupling elements, and the or at least some of the output units having a plurality of inlet ports, at least some of which are provided with coupling elements for connection each to one of a plurality of the coupling elements at the outlet ends of the cross-connector tube units, and at least some output units each having an outlet port provided with coupling elements for connection to a cannula, similar output line, whereby said at leasst some of the input units may be connected by means of its cross-connector tube units to any one of said at least some inlet ports of the output units.

The output units may comprise hollow tube-like members having inlet ports along their lengths.

The output units may incorporate separate internal channels each separately connected to an inlet port. The separate channels may have separate outputs at the outlet port of the output unit for connection, through an appropriate coupling, to similarly located separate channels in a cannula or an extension tubing leading to a cannula. In an alternative, the separate channels may be combined at or adjacent to the outlet port from the output unit. The output portion of the output unit may be longitudinally extended thereby making the use of an extension tubing unnecessary. As stated above, this extended portion may comprise separate channels each separately connected to an inlet port.

In practice, each of the plurality of output units may be connected to a different cannula, such as via extension tubing (such "extension tubing" may be the longitudinally-extended outlet portion of an output unit), or to a different lunima of the same cannula where these are of a multiple-lumen type. The combination of infusions into any one output unit is determined by the number and which of the cross-connector tubes are connected to the inlet ports of the output unit. By this means, it is possible to vary the routing of incoming infusions and also to vary with ease the number of infusions entering into any one cannula or lumina.

When an output unit contains separate channels (each connected to a respective inlet port) means may be provided to enable the possibility of the continuous flow of fluid through those channels that are not being used: "un-used" channels (i.e. those channels having no flexible cross-connection tubes, coupled to their respective inlet ports). This means thereby preventing stasis of fluid in "un-used" channels. Such means may comprise connection between in-use and un-used inlet ports or channels such that the fluid passing into an in- use port or channel may also pass into an unused port or channel. Such means may also be combined with a mechanism that prevents the flow of fluid from one in-use port or channel to another in-use port or channel.

Pressure-transducing units may be used in association with the aparatus of the present invention. Such units may comprise a hollow member provided with an entry port carrying a coupling element for connection to infusion tubing or a similar input line (such an input may comprise that from an input unit via an appropriate connection) and an outlet port provided with coupling elements for connection to a cannula, infusion extension tubing or similar output line.

The transducer unit may also be provided with a pressure sensitive mechanism disposed with respect to the lumen of the member so as to be exposed to the hydrostatic pressure therewithin and such that the pressure is transduced into another form of energy, for example, an electrical signal, and thereby translated by some other means into a pressure readout.

Separate from the pressure sensitive mechanism may be a calibration port which when opened exposes the fluid within the lumen of the transducing unit to atmospheric pressure (this enabling the pressure sensitive mechanism to be calibrated aginast a constant known pressure). Another mechanism exists to bock off the lumen distal to the calibration port when this port is opened.

The transducing unit may be provided with additional bolus ports similar to the inlet ports on output units, such additional ports in practice being used for giving intermittent boluses of drugs by syringe, but in some circumstances (e.g. when the available number of inlet ports on other output units is small) offering another lumen for infusion therapy. Such bolus ports would not be needed on transducer units which are purely used to measure intra-arterial pressure.

In one embodiment of the trasnducer unit the outlet port is provided with coupling elements for connection to an inlet port of an output unit. The outlet portion of such transducer unit may comprise a flexible tube, or in an alternative, a rigid tube.

In practice, the input fluid may be under high pressure and prevented from flowing along the transducing unit by a mechanism which blocks off the lumen of the input tubing or transducer unit. Means may be provided whereby the mechanism can be momentarily counteracted to allow flushing of the transducer unit with fluid, such as when flushing a bolus of drug out of the transducer unit into the patient.

The apparatus may incorporate stop valves associated with each input unit, each of which may comprise a device constricting a portion of each of the cross-connection tubes.

More generally, the input units and/or the output units may incorporate or have associated therewith, a device or mechanism for preventing, restricting or directing the flow of fluid, such devices comprising, for example, valves, stopcocks, or arrangements for constricting tubing and for preventing or inhibiting flow. Such devices may be positioned wherever appropriate with respect to the apparatus from the entry end of the input unit to the outlet port of the output unit. Thus, for example, the inlet ports of the output unit may each consist of a hollow stem incorporating or adjoining a resilient or flexible portion (of rubber for example) such that upon pressing on the inlet port, the resilient or flexible portion closes off that port to prevent the flow of fluid out of the port whilst a cross-connector tube is being connected. The closure of the resilient portion of the wall may be reversible under a controlled arrangement and can be linked to a catch mechanism such that continuous pressure on the part is not be needed to prevent flow when there is no cross-connector tube coupled to that port. Similar devices can be used in other portions of the apparatus.

The input units and output units may be such that they are each physically individual and may be attached, in numbers and array as required, to a support base. Alternatively, the input and output units may be fixedly attached to a support base. Alternatively, the units may be such that they may be capable of being assembled together in a multiple building fashion in numbers as required, whilst making a rigid assembly. This assembly may be attached to a support base. Alternatively, several units may be fixedly incorporated together so as to make a single device, and, in some embodiments, this device may be fixed or attached to a support base. Such a support base may additionally incorporate regions, or means, for supporting, attaching, fixing or incorporating other medical equipment or lines such as for supporting and organising wires and/or tubes from other medical devices anywhere in, on or near the patient, for example, arterial lines, ECG leads, pacing wires or temperature measuring devices or hydrostatic pressure measuring devices.

A common support base of this kind may also be used to support, affix or direct (via grooving, for example), the incoming infusion tubing and outgoing tubing to the cannula associated with the patient under therapy. The means by which the various elements of the apparatus, or the various other medical devices, may be secured on the support will be appropriate to their nature and form. for example, clip units may be used in combination with the apparatus of the invention, comprising a part for attaching the unit to the support board, and a part for holding tubes or wires. In one embodiment, the input unit may include the terminal portion of tubing of an intravenous infusion. In this case, part of the portion may be designed for fixing to the supporting base, the more remote tubing of the fixed portion then acting as the flexible cross-connector tubes. Alternatively, the supporting base may be designed such as to render fixed the part of the terminal portion of infusion tubing of a known kind.

The supporting base may be designed to fixedly secure infusion-routing equipment of known kinds, such as a sequence of three-way stop cocks either connected individually or formed in a multiple unit (e.g. a manifold), such stop cocks and units serving as a the output unit of the invention.

The output unit may incorporate special inlet ports, such as one for taking blood samples. In another example, the inlet ports may incorporate a microporous diaphragm to act as a viral and bacterial filter to decontaminate fluid passing via the inlet ports into the output unit.

The cross-connector tubes may be connected to the inlet ports on the output units by means of quick-release leak-proof couplings of the "luer-lock" type.

In an alternative, the coupling may comprise a needle, protected within and by the walls of a recess, attached to the outlet end from each cross-connection tube, adapted to pierce a rubber diaphragm over upstanding inlet ports of the output units, the walls of the recess forming a snug fit upon the ports.

By the word "rubber" used herein is included any rubber-like material such as synthetic rubbers, and appropriate elastomeric materials.

In some embodiments of the coupling, there is longitudinal extension of these walls laterally around the body of the output unit.

Means may be provided to direct the needle into the centre of the rubber diaphragm or core of an inlet port, or to stabilise or to reversibly lock the coupling on the output unit. Such means may be directly related to the coupling or, in an alternative, be provided by the support base. For example, the wall of the coupling recess or the longitudinal extension of these walls may be directed or supported by grooves, ridges or tubular elements on the output units or support board, the association of these walls with these elements may be such as to prevent rotation, or, in other embodiments, to cause or to allow rotation of the coupling on the inlet port.

In another example, the longitudinal extension may prevent rotation of the cross-connection tube coupling on the inlet port by gripping along the body of the output unit away from the axis of the coupling.

A catch mechanism may be provided between the extension and the wall of the output unit or between the extension and the support board.

In another alternative of the coupling, the male part of a coupling may comprise a hollow needle, surrounded by a protecting means, both being attached to the outlet end from each cross-connection tube, the needle being adapted to pierce a rubber sealing, e.g. a rubber diaphragm or core, over or within inlet ports, the female part of a coupling, of the output units.

The male part may be such that when it is not coupled the needle is enclosed within a protector comprising one or more longitudinal walls disposed lateral to the longitudinal axis of the needle originating adjacent at the base of the needle and extending beyond the needle point to then pass medially to cover or shield the needle point.

For coupling, means may be provided by which the longitudinal walls are moved away from the axis of the needle thereby uncovering the needle point and shaft to allow it to pierce the rubber sealing in an inlet port. Such means may include, for example, a hinge in the region of the origin of the walls, the outward and inward movement of the walls about the hinge being effected, for example, by pressure on outwardly-angled extensions of the longitudinal walls adjacent to the hinge, or, for example, by the insertion of an upstanding output unit inlet port into an inverted V-shaped recess on the outer surface of the needle point shield.

On coupling the longitudinal walls may pass laterally around the body of the output unit.

More generally, as described before, means may be provided to direct the needle into the centre of the rubber diaphragm or core of an inlet port, or to stablise or to reversibly lock the coupling on the output unit. Such means may be directly related to the coupling or, in an alternative, be provided by the support base. For example, the longitudinal walls may be directed or supported by grooves, ridges or tubular elements on the output units or support board, and the association of these walls with these elements may be such as to prevent rotation, or, in other embodiments, to cause or to allow rotation of the coupling on the inlet port.

In another example, the longitudinal walls may prevent rotation of the cross-connection tube coupling on the inlet port by gripping along the body of the output unit away from the axis of the coupling.

A catch mechanism may be provided between the longitudinal walls and the wall of the output unit or between the extension and the support board. In another example, the design of the inner surface of the longitudinal walls may be such that, on coupling, the surface forms a snug fit upon upstanding inlet ports.

The sealing means of the inlet ports may comprise rubber diaphragms covering ports disposed within the walls of the output units so that the inside walls of the units are uninterrupted or smooth, this arrangement preventing air trapping within the unit. In an alternative, the inlet ports contain a rubber core.

The inlet ports of the output units may be so arranged as to allow compatibility with other equipment carrying coupling elements similar to those on the cross-connection tubes. For example, a male part of the coupling may be carried by syringe such that drug boluses can be given by syringe.

Modifier units may be used in association with the inlet ports of the output units to enable medical equipment carrying standard coupling elements (for example a "luer-lock") to link with the inlet port. Such a modifier unit (which is placed between the standard coupling element and the coupling element of the inlet port) may comprise a hollow member incorporating at one end a coupling element similar to those on the cross-connector tubes and at the other end a standard coupling element, for example, a "luer-lock" or a syringe injection portal.

Additional cross-connector tube units, with appropriate coupling elements at each end, may be provided to connect the inlet ports of two ouput units. Switching arrangements may be provided within the output units to ensure that fluid flowing into a first output unit can be diverted to a second output unit so that no fluid flows via the outlet of the first output unit, but is diverted to and combines with the fluid flowing in the second output unit.

Alternatively, inlet-port bank units may be used in combination with the apparatus of the present invention.

The inlet-port bank unit may, by means of connection to an output unit, increase the number of infusions that can be infused into the connected output unit.

In one embodiment, the inlet-port bank comprises a blind-ending tube with inlet ports along its length. Using additional cross-connector tube, with apropriate couplings at each end, such a unit may be connected (inlet port to inlet port) to an output unit thereby increasing the number of possible infusions into that unit.

In an alternative, the inlet-port bank comprises a tube with inlet ports leading to an outlet port, the outlet port being provided with coupling elements for connection to an inlet port of an output unit. The outlet portion of such an inlet-port bank may comprise a flexible tube or, in an alternative embodiment, a rigid tube.

The inlet ports of the output units may be of such design that they can connect with and accommodate other medical equipment such as transducing devices for pressure monitoring purposes, or other monitoring equipment or devices connected to inlet ports to allow continuous or intermittent flushing of selected output units or channels therewithin.

The inlet ports on any one output unit may vary both in regard to their coupling type or their function.

In one embodiment of the invention, larger dimension input and output units may be provided than normally required for drug therapy, such larger dimension units being in practice dedicated to blood transfusion or total parenteral nutrition, for example. In addition, rapid infusion units may be used in combination with the apparatus of the invention. These rapid infusion units may comprise a single wide-bore hollow tube with an input end and an output end. The input end has an entry port carrying coupling elements for connection to infusion tubing or a similar input line. The output end has a plurality of inlet ports along its length, comparable with those on the normal output units, and an outlet port provided with coupling elements for connection to a cannula, infusion extension tubing or similar output line. The rapid infusion unit may incorporate or have associated therewith a device or mechanism for preventing, restricting or directing the flow of fluid, such as was described earlier for the normal units; and may be separate, assembled, fixedly attached, attachable etc. to a support base as described earlier for the normal units. Similarly, the rapid infusion unit may be incorporated into the terminal portion of tubing of an incoming infusion, or a portion of tubing of outgoing infusion extension tubing.

Add-on parts may be provided to change the configuration of the coupling between the flexible crossconnector tube and the inlet ports on an output unit. Such add-on parts may be directly related to the coupling or may be related to the support base. Such alteration making it impossible to connect the altered coupling of a cross-connector tube to a normal (unaltered) inlet port, and, conversely making it impossible to connect the normal (unaltered) coupling of a cross-connector tube to an altered inlet port. Similarly, several types of units may be used differing only in the shape or kind of coupling between the flexible cross-connector tube and the inlet port of the output units to be isolated and thus protect against inadvertant connection to incompatible infusions or inadvertant routing to a peripheral vein of a drug that needs to be infused into a central vein.

Parking-port units may be used in the apparatus of the present invention. These units may comprise inlet port elements such as found on output units but differing from the output units in that the parking ports do not connect with any hollow member for infusion therethrough. In practice, flexible connector-tubes outlets can be parked on these ports via their coupling (so keeping the coupling clean) until they are required, when they are un,coupled and transferred to the inlet port of an output unit.

Coding by colour or marking may be used to enhance identification of routings and combination of infusions on the apparatus of the invention. Thus, for example, the entry and outlet ends of the input units, the inlet ports and the outlet of the output units may all be coded. In addition, this may be associated with further coding of infusion tubing both incoming and outgoing.

Again coding of the input units and output units may also be affected by differences in the size and/or shape of the units, or parts of the units, and/or by incorporating areas where coding legends can be written.

Means may be provided by which the date of first use of the apparatus may be ascertained. Such means may be attached or attachable to the apparatus and may include, for example, a design of the numbers one to thirty-one inclusive, such that the date in the month may be marked (by piercing with a needle, for example).

Means may be provided by which the duration of use of any selected part of the apparatus of the present invention can be determined. Such a means, which may be separate from or incorporated into any part of the apparatus, may comprise a time-duration indicator capable of changing colour from a first colour state once activated to other colour states over known periods so that the period since activation can be determined by the colour.

The indicator may function on the basis of a chemical reaction such that it changes colour as the reaction proceeds.

The reaction may be initiated by exposing the indicator to a source of energy such as light or heat (for example by peeling away a protectiove cover), or it may be initiated by exposure to another chemical either present in atmospheric air (e.g. carbon dioxide, oxygen or nitrogen) or present in another part of the indicator means. Such an indicator may include a catalyst or enzyme.

Coding, labelling, dating or duration of use indicators, which may be permanent or of an "add-on" nature may be such that they can be incorporated at any position in infusion apparatus from the infusion source bag to the cannula. Tubing may be such that labels may be placed into appropriately located slots in the walls thereof and/or may incorporate labelled tags which are part of the plastic or rubber parts of tubing. Thus, one such tag may be placed near the infusion source bag and one placed near the end of the infusion tubing. Such tags can be used to locate the tubing on a support.

The apparatus may be protected from infection by means of an overall transparent cover. In general, covers may be used to protect selected areas of the apparatus of the present invention, for example, the input unit attachment area may be covered along its length by a transparaent cover which helps to protect and secure the input units, Such a cover may also incorporate label slots disposed over each input unit.

The apparatus may be capable of attachment to the patient or the patients bed or equipment associated therewith.

In one embodiment of the invention, a two-part coupling device may be provided comprising male and female parts having passageways therethrough spaced around, or lateral to, the axis of the coupling for suitable interconnection upon "a plug in" jointing thereof. Inlet tubes to each passageway of the "plug" coupling may be provided at their inlet ends with coupling elements each capable of connecting to an outlet of the output units, the outputs of the plug coupling in turn being connected to a multiple lumen extension tube, or to a plurality of lumina within a single cannula. By this means, the individual lumina or tubes within the cannula can be kept associated with each other outside the body, only being separated adjacent to the apparatus of the present invention. Alternatively, the inlet tubes to each passageway of the "plug" coupling may each be incorporated into and continuous with the outlets of respective output units or the outlets of respective internal channels within a multiple-lumen output unit.

Any of the couplings or connections associated with the apparatus of the present invention may be provided with means to prevent uncoupling of the parts once first coupling has occurred. Such a means may be directly related to the coupling or connection, for example a jaw mechanism located on and around a female part of the luer-lock may open as the male part is coupled between the jaws, this then closing over the proximal part of the male part so as to prevent it from being uncoupled. In an alternative the means preventing uncoupling may be provided by the support base .

Means may be provided, which may be separate from the input units and the output units and any support base, for collecting together and supporting in an organised fashion, incoming and outgoing lines (for example, infusion tubing or electric wires) from the apparatus of the present invention. Such means may comprise, for example, a relatively large diameter flexible tube capable of containing all such incoming or outgoing lines and openable along its length to allow inspection of the lines. In an alternative, such means may comprise a grooved plastic bar, the lines being disposed within the grooving. Means may also be provided to collect together selected flexible cross-connection tubes. Such means may be combined with or separate from any of the input units, the flexible connector tubes, the output units or the support board.

In order that the invention may be more readily understood, embodiments thereof will now be described by way of example only with reference to the accompanying schematic drawings in which:-

Figure 1 is a side view of one embodiment of apparatus according to the invention;

Figure 2 is a plan view of the apparatus of Figure 1;

Figure 3 is a view of one form of output unit;

Figure 4 illustrates an output connection of the unit of Figure 3;

Figure 5 is a view of another form of output unit;

Figure 6 shows yet another form of output unit;

Figure 7 shows an alternative form of output unit to that of Figure 6.

Figures 8A and B are representations of the operation of the output unit of Figure 7;

Figures 9A and B represent an alternative arrangement of that of Figure 8 of an output unit and its operation;

Figures 10A, B and C illustrate fluid control means for use in the apparatus of invention;

Figure 11 illustrates a mounting arrangement for units in accordance with the invention;

Figure 12 shows an alternative dispostion of an output unit on the support base;

Figure 13 shows an multiple assembly of units in accordance with the invention;

Figure 14 illustrates a support base with attachment means for medical devices;

Figure 15 illustrates another arrangement of units on a support base;

Figures 16A and B illustrate alternative attachments of an input line to a support base;

Figure 17 illustrates an embodiment of output unit;

Figures 18 and 19 illustrate one form of coupling to an inlet port of an output unit;

Figure 20 illustrates a cross-connector unit;

Figure 21 illustrates a still further form of output unit;

Figure 22 illustrates a form of cross-connector tube between two output units;

Figure 23 illustrates the connectability of an inlet port of an output unit to a medical device;

Figure 24 illustrates a rapid infusion unit for use with the present invention;

Figure 25 and illustrates an alternative coupling unit between an inlet port and a cross-connector unit;

Figure 26 illustrates labelling facilities with the apparatus of the invention;

Figure 27 illustrates tubing and line support means;

Figure 28 illustrates transducer units for use in association with the apparatus;

Figure 29 illustrates a transducer unit for use with the apparatus of the present invention;

Figure 30 illustrates a convertor coupling element and its use;

Figure 31 illustrates parking ports;

Figure 32 illustrates one form of irreversible locking coupling; and

Figure 33 illustrates a multilumen plug connector.

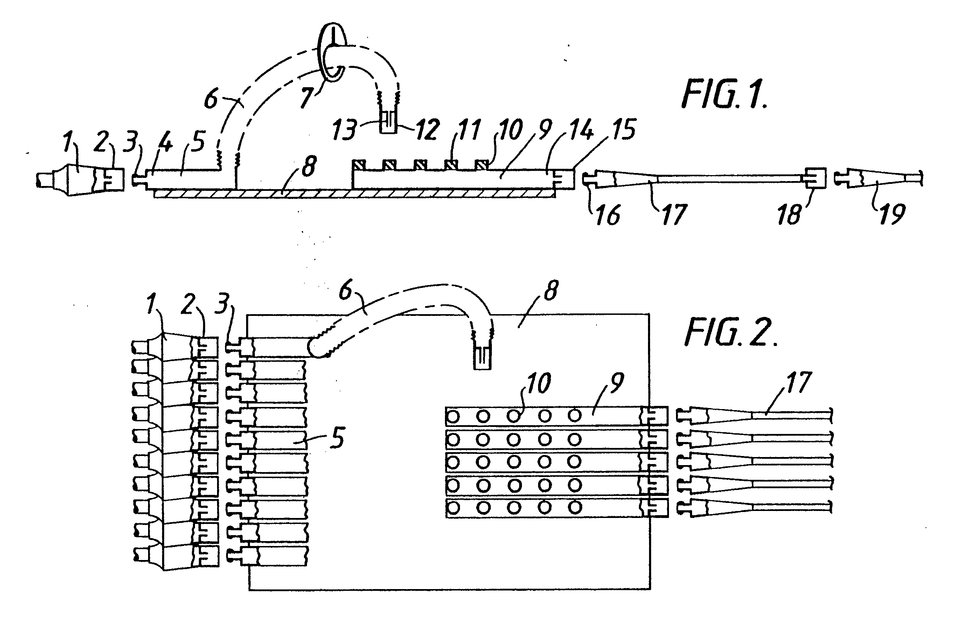

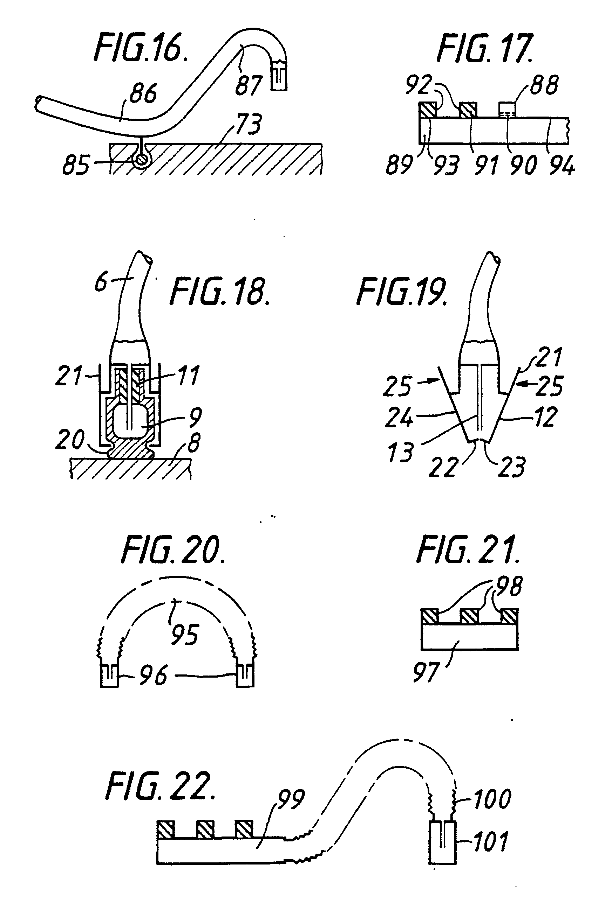

Referring now to Figures 1 and 2 of the drawings taken in association with Figures 18 and 19 it will be seen that a plurality of incoming intravenous infusion tubes 1 are provided at the outlet ends with male elements of a luer-lock 2 which in use are adapted to connect with female elements 3 of the luer-lock mounted on the entry ports 4 of input units 5. Flexible cross-connector tubes 6 are disposed at the outlets from all of the input units, each of said cross-connector tubes being provided with a crimping device 7 of known kind for restricting or preventing flow therethrough.

The plurality of input units 5 are mounted upon a support board 8, as are a plurality of output units 9 each of which has along its length a plurality of inlet ports 10 containing a protective rubber core 11. Each of the cross-connector tubes from the outlet of each of the input units 5 is provided at their outlet ends (as shown in Figures 18 and 19) with a connector comprising a hollow needle 13 whose sharp tip is shielded by the medial extensions 22, 23 of two longitudinal walls 12, 24. In use, pressure on the arms 21 in the direction shown by arrows 25 moves the longitudinal walls and needle-point shield away from the needle-point. The coupling can then take up position on the inlet port with the inner surface of the longitudinal walls forming a snug fit upon the inlet ports and output unit, with the needle 13 penetrating and traversing the rubber core to allow the flow of liquid therethrough. A catch mechanism 20 is provided between the longitudinal walls and the output unit. Pressure on the arms 21 releases the catch. Each cross-connection tube 6 is able to be coupled to any of the ports 10 of any of the output units.

The outlet 14 from each of the output units is provided with the male elements 15 of a "luer-lock" for connection to a corresponding female portion of a "luer-lock" disposed at the inlet end of a length of tubing 17 which in turn connects at its outlet end 18 via a "luer-lock" to an injection cannula 19 associated with a patient undergoing therapy.

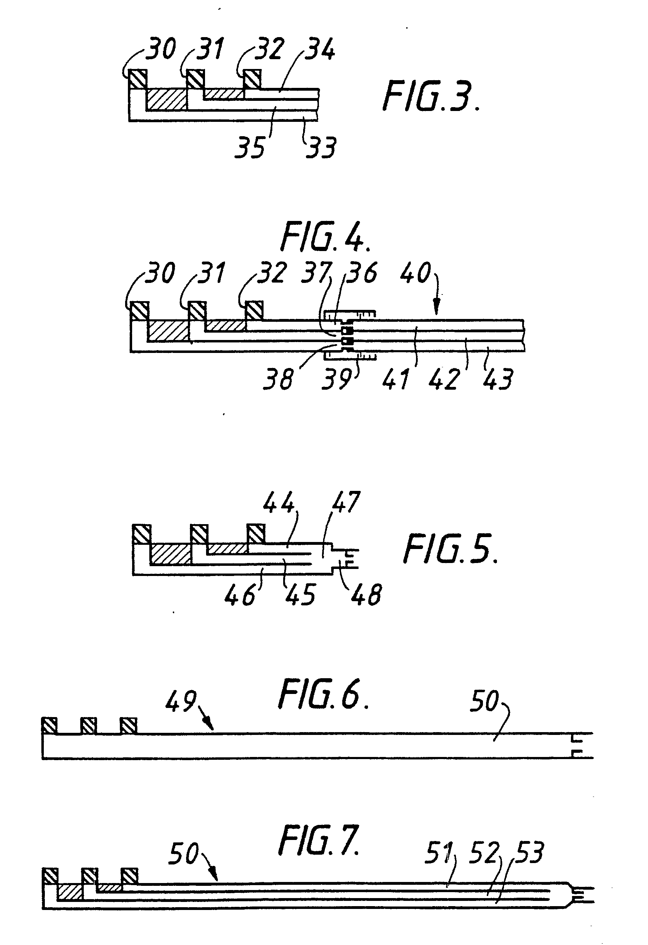

In Figure 3 there is illustrated an output unit having inlet ports 30, 31, 32 and separate internal channels 33, 34, 35 each separately connected to one of the inlet ports. As can be seen in Figure 4 the separate channels may have separate outputs 36, 37, 38 at the outlet port of the output unit for connection via a coupling 39 to an output line 40 having similar separate channels 41, 42, 43. Figure 5 shows a similar arrangement where the separate channels 44, 45, 46 are combined 47 adjacent the outlet port 48 from the output unit.

As can be seen in Figure 6 an output unit 49 can have a longitudinally extended output portion 50 to dispense with the need for extension tubing in association therewith. Figure 7 shows a similar arrangement wherein the elongate extended portion 50 of the output unit includes separate channels 51, 52, 53 each separately connecting to an inlet port.

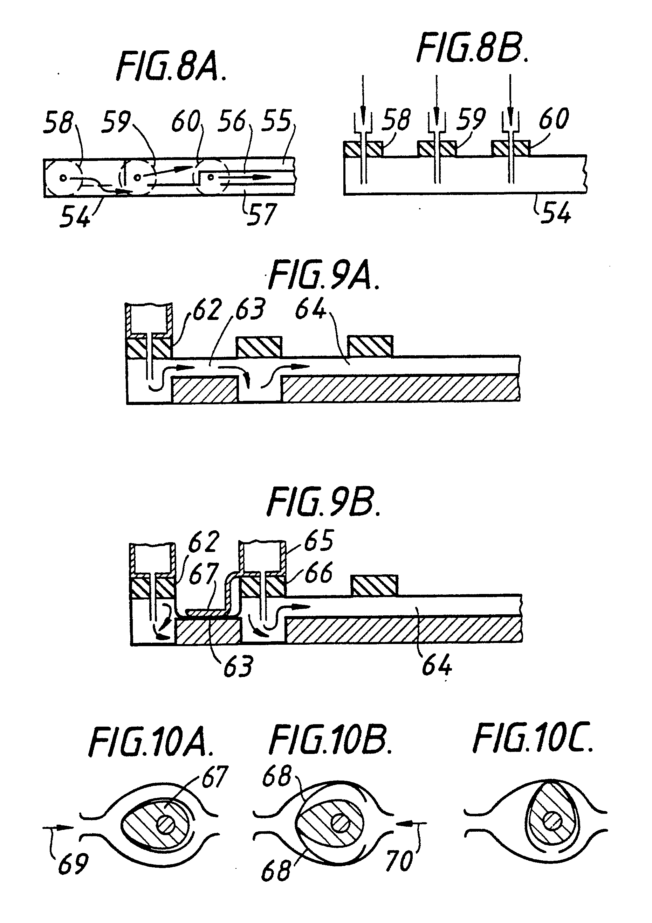

Figures 8A and 8B illustrate an arrangement of an output unit 54 having a multiplicity of channels 55, 56, 57 coupled at the inlet ports 58, 59, 60 thereto such that a continuous flow of fluid may pass through unused channels to maintain use of all channels.

Figures 9A and 9B illustrate an alternative arrangement of output unit 61, incorporating a mechanism preventing the flow of fluid from one in-use port 62 or channel 63 to another. Thus, when inlet port 62 is coupled fluid flows into channels 63 and 64. However, the coupling member 65 then used with port 66 has a side arm 67 which collapses the flexible wall of channel 63 and prevents fluid from inlet port 62 reaching inlet port 66.

Figure 10 illustrates the use of a valve 67 for restricting and controlling the flow of fluid within apparatus according to the invention. As can be seen the valve has flexible or hinged wings 68 and can be in an open position for fluid to flow in the directionof arrow 69 and closed for flow in the direction of arrow 70 and also turned as in Figure 10C to flow from both directions.

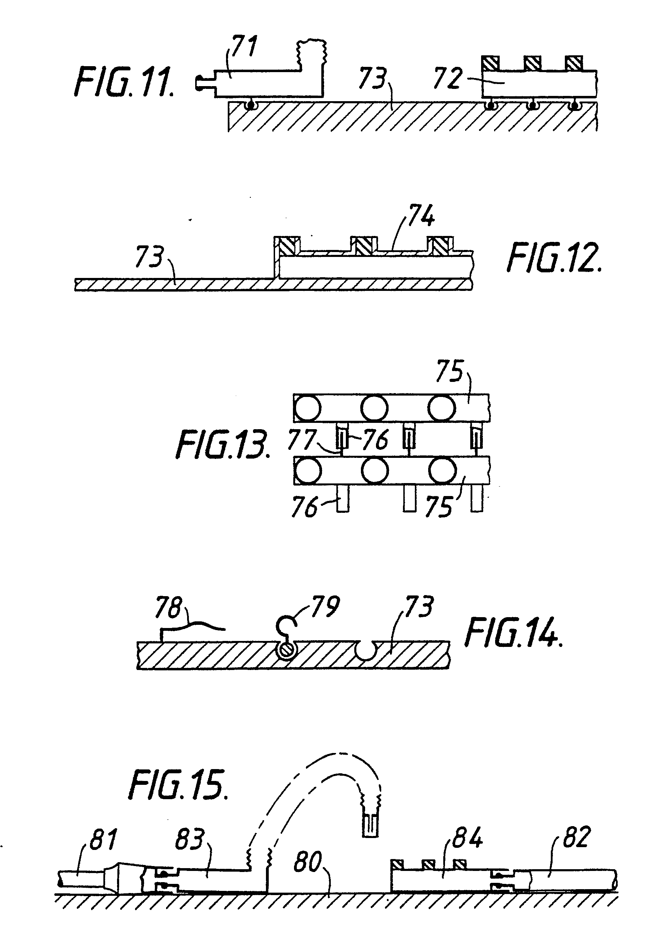

As can be seen from Figure 11, in one embodiment of the invention units 71, 71 of the apparatus can be physically separable items or groups of items which may be attached to a support base 73. In an alternative arrangement the input units and output units may be fixedly attached to the support base or indeed be integral therewith as is shown at 74 in the Figure 12. In yet another arrangement, as is shown in Figure 13, the units 75 may be adapted by clips 76, 77 for assembly together in a multiple building fashion.

Figure 14 shows clips 78, 79 attached to the support base 73 for carrying medical devices.

Figure 15 illustrates an extended support base 80 arranged to support and direct incoming and outgoing lines 81, 82 to and from input units 83, and output units 84, or other parts of the assembly.

Figure 16 illustrates attachment means 85 on the support base 73 for terminal portions of tubing 86 of an intravenous infusion line, part of which can be arranged to serve as the flexible cross connector tube unit 87.

Figure 17 illustrates the provision in an inlet port 88 of an output unit 89 with a micro-porous diaphragm 90 acting as a decontaminating filter. Additionally it illustrates that such a diaphragm, or alterantively rubber plugs 91, within the ports 92, can be disposed such that their inner surfaces 93 are disposed at or adjacent the inner wall 94 of the output unit 89 such that the wall 94 have no discontinuities.

Figure 20 illustrates a cross- connector tube unit 95 adapted by means of coupling elements 96 at each end for cross connection between inlet ports of two output units.

Figure 21 illustrates an output unit 97 having no outlet port, which is capable however of connection to other output units by means of its inlet ports 98 via a cross connector tube unit of the kind shown in Figure 20.

Figure 22 illustrates a related modification of output unit 99, in which the outlet 100 thereof comprises a flexible extended tube having appropriate coupling elements 101 to enable that outlet to be connected to the inlet port of another outlet unit.

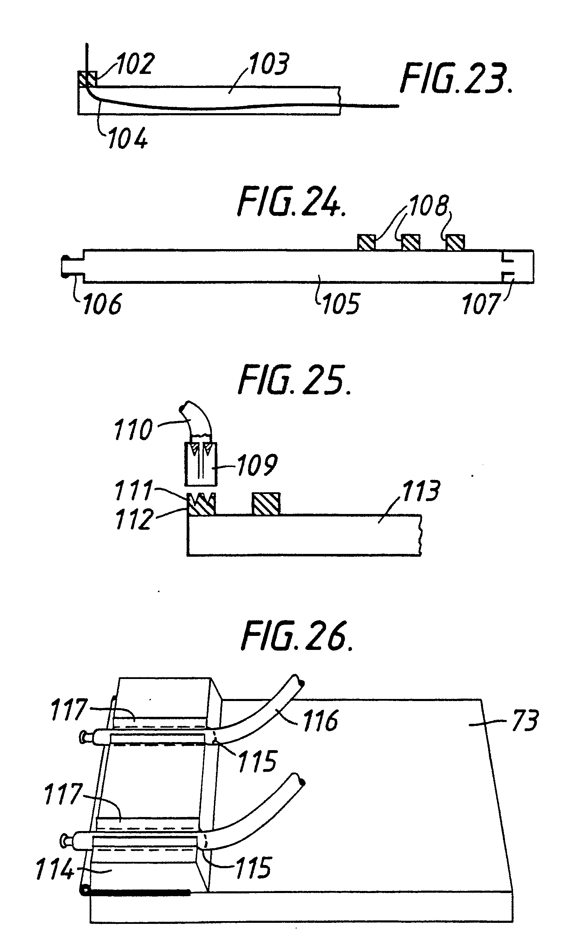

Figure 23 illustrates an output unit having an inlet port 102 of an output unit 103 adapted to receive a medical device other than a cross connecting tube unit. In this case the device is an electrical heart pacing wire 104.

In addition to the inlet and outlet units as hereinabove described, the apparatus of the present invention may include a rapid infusion unit as illustrated in Figure 24 comprising a relatively wide bore single hollow tubular member 105 having a straight through passageway between an input entry port 106, and an output exit port 107, the output end carrying a plurality of inlet ports 108 connectable, in the usual way of the invention, to cross connector tube units or other appropriate lines.

Figure 25 illustrates an embodiment of the invention in which the configuration of the coupling element 109 of a cross connector tube unit 110, and the associated coupling element 111 of an inlet port 112 of an output unit 113 are so configured as to be only capable of connection with each other, or with coupling elements similarly configured, thereby preventing cross connection between units carrying incompatible fluids.

Parts of the apparatus, such as the support base 73, may, as illustrated in Figure 26, carry labelling means. In the instance illustrated a hinged transparent cover 114 attached to the support base may be provided with grooves 115 for carrying input lines 116, for example, and may be provided with slots for appropriate identification labels 117, or chronological labels concerning date of first use or time of use may be disposed in association with the appropiate line.

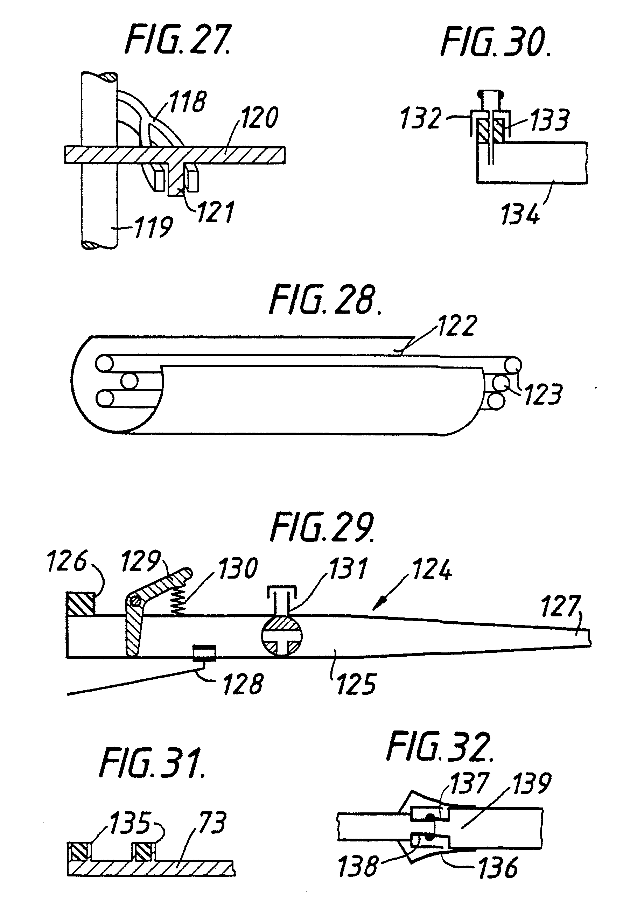

The apparatus of the present invention, together with its support base may be associated with the patient or his bed assembly. Thus, in Figure 27 an arrangement is illustrated whereby by means of an arm 118 attached to a drip stand 119 forming part of the bed equipment of a patient. A support base 120 is carried by means of a pendant flange 121 in association therewith. To maintain tidiness and convenience of the apparatus in accordance with the present invention means may be provided, such as a wrap around semi-tubular member 122 illustrated in Figure 28 for linking together the various input and output lines 123 of the apparatus.

Figure 29 illustrates a of pressure-transducing units 124 which may be included in the apparatus of the present invention. The unit comprises a hollow member 125 provided with an inlet port 126 for connection to a source of high pressure fluid and an outlet 127 for connection to a cannula, infusion extension tubing or similar output line. Illustrated in Figure 29, as typical of the form of transducer units that can be utilised, a pressure/electric transducer 128 for sensing blood pressure, when the hollow member is connected to an artery of a patient for example. A valve flush unit 129 by means of which the connected high pressure fluid normally held back by the flush unit valve can be released against the urging of a spring 130 to allow flushing from the unit of a patient's artery, for example. An air valve 131 for pressure calibration is included.

As previously mentioned the coupling elements with respect to the inlet ports of the output units of the apparatus of the present invention may be adapted to receive inputs other than cross connection tube units. Figure 30 illustrates an appropriate modifying coupling unit 132 which, in the instance illustrated, would enable a "luer-lock" connection from a medical device to be made to an inlet port 138 of an output unit 134.

The apparatus may include elements which can be called "parking ports" which comprise blank inlet ports not connected to output units. Two such ports 135 are illustrated in Figure 31 mounted on support base 73. Such devices enable the output ends of the connection tube units to be located keeping them clean and held down.

Figure 32 illustrates a coupling which prevents ready uncoupling once a first connection has occurred. In this instance a mechanism of arms 136 are located on and around a female part 137 of a "luer-lock" arrangement on n inlet port 139 as the male part 138 is coupled between the arms 136. These close over an adjacent part of a male part 138 so as to prevent it from being uncoupled.

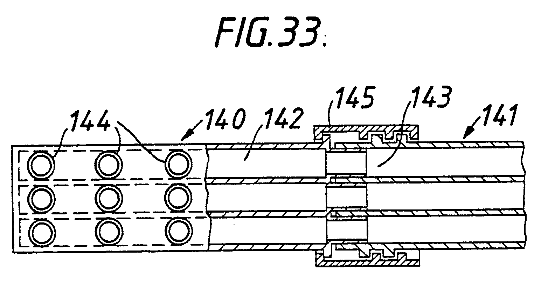

Figure 33 illustrates a two part coupling device between a multi-channel male member 140 (having inlet ports 144) and a multi-channel female member 141, corresponding channels 142, 143 in each part connecting with each other upon appropriate snap engagement of the coupling together via the snap-in connector collar 145 of the female part.

By means of the invention we have provided apparatus capable of multiple interconnection in patient therapy between a plurality of drug infusion and monitoring equipment in a safe and convenient form.

It is to be understood that the foregoing is merely exemplary of intravenous apparatus in accordance with the invention and that modifications can readily be made thereto without departing from the true scope of the invention.


**Claims**

1. Apparatus for intravascular infusion comprising an assembly of a plurality of hollow input connection units, and at least one hollow output connection unit, the input units comprising hollow members each having an entry extending from infusion tubing or a similar input line, and leading via a flexible cross-connector tube unit terminating in an output end carrying a coupling element, the at least one output unit comprising a hollow member having at least one inlet port, such inlet port being provided with coupling elements for connection to the coupling elements at the outlet ends of the cross-connector units and the or at least one output unit having an outlet port provided with coupling elements for connection to a cannula, or similar output line whereby the input units may be connected by means of their cross-connector units to the at least one inlet port of the at least one output unit.

2. Apparatus as claimed in Claim 1 comprising an assembly of a plurality of hollow input connection units and a plurality of hollow output connection units, at least some of the input units having an entry port carrying coupling elements for connection to infusion tubing or a similar input line, and at least some of said input connection units linking directly or indirectly to a flexible cross-connector unit, at least some of the cross-connector tube units terminating in coupling elements, and at least some of the output units having a plurality of inlet ports at least some of which are provided with coupling elements for connection each to one of a plurality of the coupling elements at the outlet ends of the cross-connector tube units and at least some output units each having an outlet port provided with coupling elements for connection to an outlet line whereby said at least some of the inlet units may be connected by means of the cross-connector tube units to any one of the said at least some inlet ports of the output units.

3. Apparatus as claimed in Claim 1 or 2 wherein the or at least one of the output units incorporate separate internal channels each separately connected to an inlet port.

4. Apparatus as claimed in Claim 3 wherein the separate channels have separate outputs at the outlet port of the output units for connection, via an appropriate coupling, to similarly located separate channels in an appropriate output line.

5. Apparatus as claimed in Claim 3 wherein the separate channels are combined at or adjacent the outlet port from the output unit.

6. Apparatus as claimed in any one of the preceding claims wherein the or at least one of the output units has a longitudinally extended output portion so as to dispense with the need for extension tubing thereto.

7. Apparatus as claimed in Claim 6 wherein said extended portion includes separate channels each separately connecting to an inlet port.

8. Apparatus as claimed in any one of the preceding claims wherein the or at least one of the output units incorporates separate internal channels each separately connected to an inlet port, and including means provided to allow continuous flow of fluid through those unused channels of the separate channels which in a particular operation of the apparatus would not otherwise be used for fluid for infusion purposes.

9. Apparatus as claimed in Claim 8 wherein said means provided to enable continuous flow of fluid comprises connector means between in-use and unused inlet ports or channels whereby the fluid passing into an in-use port or channel also passes into an unused port or channel.

10. Apparatus as claimed in Claim 9 wherein said means is combined with mechanism preventing, in operation, the flow of fluid from one in-use port or channel to another in-use port or channel.

11. Apparatus as claimed in any one of the preceding claims wherein at least some of the input units and/or the or at least some of the output units are physically separable individual items or groups of items.

12. Apparatus as claimed in any one of the preceding claims wherein at least some of the input units and the or at least some of the output units are adapted such as to be capable of assembly together in a multiple building fashion in numbers and array as required such as to form a rigid assembly.

13. Apparatus as claimed in any one of the preceding claims wherein the assembly is attached to a support base.

14. Apparatus as claimed in Claim 13 wherein the support base is provided with means for supporting or incorporating medical devices additional to the input units and the at least one output unit.

15. Apparatus as claimed in any one of Claims 13 or 14 wherein the support base, in addition to supporting the input units and the at least one output unit, includes means for supporting or directing incoming and outgoing lines to and from the input units and the output units and other parts of the assembly.

16. Apparatus as claimed in any one of the preceding claims wherein at least one of the input units includes a terminal portion of tubing of an intravenous infusion line, a part thereof being arranged for attachment to the support base, and a further part thereof serving as the flexible cross-connector tube unit.

17. Apparatus as claimed in any one of the preceding claims wherein at least one of the inlet ports incorporates a microporous diaphragm acting as a decontaminating filter.

18. Apparatus as claimed in any one of the preceding claims wherein at least one cross-connector tube unit is connectable to the or at least one of the inlet ports on the or at least one of the output units by means of releasably leakproof couplings.

19. Apparatus as claimed in any one of the preceding claims wherein at least one of the cross-connector tube units is connectable to the or at least one of the inlet ports on the or at least one of the output units by means of a coupling having a male part attached to the cross-connector tube units comprising a hollow needle surrounded by protecting means, and a female part attached to the or at least one of the inlet ports and comprising a rubber inlet port sealing member.

21. Apparatus as claimed in Claim 19 in which the protecting means surrounding the hollow needle comprises at least one longitudinal wall disposed lateral to the longitudinal axis of the needle and extending beyond and sheilding the point of the needle and being capable of substantial separable movement from the needle to enable the needle to engage and pierce the rubber sealing member at the inlet port, the inlet port having engagement means for engaging the at least one longitudinal wall.

21. Apparatus as claimed in any one of Claims 1 to 18 wherein at least one of the cross-connector tube units is connectable to the or at least one of the inlet ports by means of a coupling comprising a tubular member defining a recess within which is located a hollow needle, the inlet port having a rubber sealing member arranged in use to be pierced by the hollow needle and the internal walls of the tubular member being arranged to fit about the inlet port.

22. Apparatus as claimed in any one of the preceding claims wherein couplings between units of the assembly are provided with a catch mechanism.

23. Apparatus as claimed in any one of the preceding claims including a rubber sealing member protecting the or at least one of the inlet ports is arranged such that its inner surfaces are disposed at or adjacent the internal walls of the output unit such that said walls are uninterrupted.

24. Apparatus as claimed in any one of the preceding claims including a plurality of output units wherein there is included additional cross-connector tube units with appropriate coupling elements at each end to enable the interconnection of inlet ports of separate output units.

25. Apparatus as claimed in Claim 24 including output units provided with no outlet ports, and capable of connection to other output units via their inlet ports and said additional cross-connector tube units.

26. Apparatus as claimed in any one of the preceding claims including a plurality of output units wherein at least one output unit has an outlet capable of direct or indirect coupling to an inlet port of another output unit.

27. Apparatus as claimed in any one of the preceding claims including a plurality of output units wherein the or at least one of the inlet ports are arranged to be capable of connection with medical devices other than cross-connector tube units, via couplings therebetween.

28. Apparatus as claimed in any one of the preceding Claims including at least one rapid infusion unit comprising a single hollow tubular member having an input end and an output end, the input end having an entry port carrying coupling elements for connection to an input line, and the output end carrying a plurality of inlet ports along its length connectable to cross-connector tube units and other input lines, and an outlet port provided with appropriate coupling elements for relevant output lines.

29. Apparatus as claimed in any one of the preceding claims wherein the coupling elements of at least one cross-connector tube units are configured or are provided with convertor elements configured such that they are only capable of connection with elements of other parts of the apparatus similarly configured.

30. Apparatus as claimed in any one of the preceding claims wherein parts of the apparatus are provided with identification coding means.

31. Apparatus as claimed in any one of the preceding claims incorporating means giving chronological information.

32. Apparatus as claimed in any one of the preceding claims incorporating means for attachment thereof to a patient or his bed equipment.

33. Apparatus as claimed in any one of the preceding claims including a protective cover therefor.

34. Apparatus as claimed in any one of the preceding claims including a two part coupling device, the two part coupling device comprising male and female parts each having passageways therethrough spaced thereabout, the coupling device being arranged to provide appropriate interconnection between respective passageways on forming the coupling, the coupling being provided with inlet and outlet tubing to each passageway having at their ends coupling elements for connection to input and output lines respectively, enabling the coupling to be connected on the input or the output side of the apparatus.

35. Apparatus as claimed in any one of the preceding claims including means for collecting together and supporting lines to and from the apparatus.

36. Apparatus as claimed in any one of the preceding claims including a transducer unit comprising a hollow member connectable via a shut off valve at an inlet end to high pressure flushing fluid and at an outlet end to an output line to a patient, and having associated therewith patient pressure sensitive transducer means for providing a pressure related signal.

37. Apparatus as claimed in any one of the preceding claims including blank units carrying members equivalent to output unit inlet ports for connection with the outlets of unused cross-connector tube units.

38. Apparatus as claimed in any one of the preceding claims including coupling elements for parts thereof arranged to inhibit ready disconnection after coupling between the elements has taken place.

39. Apparatus as claimed in any one of the preceding claims including fluid control mechanism serving to prevent, restrict, control and/or direct the flow of fluid through or within the apparatus.

FIG.1.

FIG.2.

EP 0 405 907 A1

FIG.3.

30 31 32 34

35 33

FIG.4.

30 31 32 37 36 40

38 39 41 42 43

FIG.5.

44 47

46 45 48

FIG.6.

49 50

FIG.7.

50 51 52 53

14

## FIG.8A.

58 59 60 56 55

54 57

## FIG.8B.

58 59 60

54

## FIG.9A.

62 63 64

## FIG.9B.

62 65

67 66

63 64

## FIG.10A.

67

69

## FIG.10B.

68

68 70

## FIG.10C.

FIG.11.

FIG.12.

FIG.13.

FIG.14.

FIG.15.

EP 0 405 907 A1

**FIG.16.**

86  87
73
85

**FIG.17.**

92  88
89  93  91  90  94

6

**FIG.18.**

21  11
20  9
8

**FIG.19.**

25  21
25
24  12
13
22  23

**FIG.20.**

95
96

**FIG.21.**

98
97

**FIG.22.**

99  100
101

17

FIG.23.

FIG.24.

FIG.25.

FIG.26.

## FIG. 27.

118  120

119  121

## FIG. 30.

132  133

134

## FIG. 28.

122

123

## FIG. 29.

126  129  130  131  124  127

128  125

## FIG. 31.

135  73

## FIG. 32.

137  139

138  136

FIG.33.

| DOCUMENTS CONSIDERED TO BE RELEVANT | | | EP 90306964.9 |
|---|---|---|---|
| Category | Citation of document with indication, where appropriate, of relevant passages | Relevant to claim | CLASSIFICATION OF THE APPLICATION (Int. Cl.⁵) |
| X | US - A - 2 954 028 <br> (R.C.SMITH) <br> * Totality; especially fig. 1-3,7; column 3, lines 59-62; column 5, lines 27-32 * | 1,2, 11,13- 16,18, 27,35, 39 | A 61 M 5/14 |
| A | -- | 12 | |
| A | WO - A1 - 88/03 815 <br> (R. BISCHOF) <br> * Fig.; page 4, line 32 - page 5, line 32; page 7, lines 21-23 * | 1 | |
| A | -- <br> GB - A - 2 020 554 <br> (P. SEIDEL) <br> * Totality * <br> ---- | 1 | |
| | | | TECHNICAL FIELDS SEARCHED (Int. Cl.⁵) |
| | | | A 61 M |

The present search report has been drawn up for all claims

| Place of search <br> VIENNA | Date of completion of the search <br> 04-10-1990 | Examiner <br> LUDWIG |
|---|---|---|